# EUROPEAN PATENT APPLICATION

(11) **EP 4 148 147 A1**
(43) Date of publication of application: **15.03.2023**
(21) Application number: 21196148.7
(22) Date of filing: 10.09.2021
(51) Int. Cl.: C12Q 1/6883, A01K 67/027, A61K 38/00

(54) **MEANS AND METHOD FOR THE DIAGNOSIS AND TREATMENT OF AUTISM SPECTRUM DISORDERS BASED ON THE DETECTION AND MODULATION OF A DEUBIQUITINASE**

(71) Applicant: Universitätsmedizin der Johannes Gutenberg-Universität Mainz, 55131 Mainz (DE)
(72) Inventor: SCHMEISSER, Michael, 55131 Mainz (DE); WAISMAN, Ari, 50678 Köln (DE)
(74) Representative: Kuttenkeuler, David

(57) **Abstract**

The invention is based on the detection of the involvement of the K63-specific deubiquitinase CYLD in the manifestation of autism spectrum disorder in a mouse model. The invention therefore provides methods for the diagnosis of such a disorder as well as methods for the development of new autism diagnostics. Further provided are means and methods for use in therapeutically modulating any manifestation of an autism spectrum disorder, or intellectual disability (ID), in a mammal or associated neuropsychiatric manifestations.

## Description

### FIELD OF THE INVENTION

The invention is based on the detection of the involvement of the K63-specific deubiquitinase CYLD in the manifestation of autism spectrum disorder in a mouse model. The invention therefore provides methods for the diagnosis of such a disorder as well as methods for the development of new autism diagnostics. Further provided are means and methods for use in therapeutically modulating any manifestation of an autism spectrum disorder in a mammal or associated neuropsychiatric manifestations.

### DESCRIPTION

Autism Spectrum Disorder (ASD) covers a broad spectrum of neurocognitive and social developmental delays with typical onset before 3 years of age including Autistic Disorder, Pervasive Developmental Disorder-Not Otherwise Specified and Asperger's Disorder as sub classified in the Diagnostic and Statistical Manual of Psychiatric Disorders, 4th edition, Text Revision (DSM-IV-TR). Prevalence of ASD has been increasing during last decades, and current estimation is 1 in 90 (Kogan, M. D., et al. Prevalence of parent-reported diagnosis of autism spectrum disorder among children in the US, 2007. Pediatrics 124, 1395-1403 (2009)) to 3.7 in 10002. There are waiting lists for evaluation by most centers with expertise, and despite the progress made in adopting instruments such as the Autism Diagnostic Interview-Revised (ADI-R) and the Autism Diagnostic Observation Schedule (ADOS) there remains significant debate regarding the prognostic value and accuracy of existing instruments. Thus, improved diagnostic and therapeutic approaches are needed.

CYLD is a deubiquitinating (DUB) enzyme first identified as being mutated in familial cylindromatosis, an autosomal dominant genetic predisposition to multiple tumors, called cylindromas (1, 2). CYLD is located in the cytoplasm and its C-terminal catalytic domain mediates the cleavage of tetra-ubiquitin to tri-, di-, and mono-ubiquitin, with a preference for Lys63- or Meti-linked polyubiquitin chains from several substrates (3). The N-terminus comprises three cytoskeletal-associated protein-glycine-rich (CAP-Gly) domains, which can bind microtubules facilitating cytoskeleton formation (4).

Although CYLD is highly expressed in the brain (1, 5), surprisingly only few data are available on its role there. Specifically, CYLD was identified in the postsynaptic density (PSD) as detected by mass spectrometry analysis, immunoblotting and immuno-electron microscopy (6, 7). In addition, immunogold labelling of dissociated hippocampal cultures under basal and depolarizing conditions, showed that CYLD expression significantly increases at PSDs upon neuronal depolarization (7). It was further shown that CYLD recruitment to the PSD is dependent on the activation of Ca2+/calmodulin-dependent protein kinase II (CaMKII) upon membrane depolarization and N-methyl-D-aspartate (NMDA) receptor activation (8). Thus, CYLD is recruited to synapses in an activity-dependent manner and involved in the regulation of several signaling pathways. In addition, CYLD was shown to control synapse organization by localizing and compartmentalizing specific synapse targets by deubiquitinating PSD-95, that functions as a major scaffold protein organizing the structure of the PSD (9). Moreover, CYLD was shown to positively regulate dendritic growth by promoting both α-tubulin acetylation in mouse hippocampal neurons and through the interaction of its first CAP-Gly domain with microtubules (10).

Recently, several neuropsychiatric disorders such as autism spectrum disorder (ASD) have been linked to molecular changes in synaptic connections. Interestingly, the proteome analysis of striatal synaptosomes from two mutant lines for Shank3, a major ASD candidate gene, revealed that the amount of synaptic CYLD is significantly increased and reduced in the striatum of Shank3 overexpressing and Shank3 deficient mice, respectively (11, 12).

In this study the inventors investigated the role of CYLD in synaptic dysfunction related to ASD. The inventors show that Cyld-/- mice exhibit major autism-like phenotypes including impaired social communication, increased repetitive behavior and cognitive dysfunction. In addition, the inventors associate the behavioral phenotypes with reduced basal synaptic transmission, impaired network excitability and reduced long-term potentiation (LTP) in Cyld-/- hippocampus. Further, the presence of K63-linked polyubiquitin substrates at the synapse and the abundance of CYLD in the PSD fraction suggest that CYLD is a primary DUB for multiple synaptic proteins. Based on the inventor's data, the inventors suggest that CYLD controls mTOR signaling, and the regulation of autophagic processes. Indeed, the deletion of Cyld resulted in increased hippocampal mTOR activity correlating with decreased levels of the autophagy marker LC3B-II within hippocampal synaptosomes.

Thus, it is an objection of the invention to provide strategies for the diagnosis and/or treatment of autism spectrum disorder as well as the modulation of associated neuropsychiatric manifestation.

### BRIEF DESCRIPTION OF THE INVENTION

Generally, and by way of brief description, the main aspects of the present invention can be described as follows:

In **a first aspect,** the invention pertains to a method for diagnosing the presence or an increased risk of developing an autism spectrum disorder, or intellectual disability (ID), in a subject, the method comprising: obtaining a nucleic acid from a tissue or body fluid sample obtained from a subject; conducting an assay to identify whether there is a variant sequence, or a plurality of variant sequences, in the subject's nucleic acid; for each variant detected, determining if the variant is a known variant associated with an autism spectrum disorder, or intellectual disability (ID), or a previously undescribed variant; if the variant is a previously undescribed variant, determining if the variant is expected to have a deleterious effect on at least one of gene expression and/or protein function; and diagnosing the presence or an increased risk of developing the autism spectrum disorder based on the variant sequence or the plurality of variant sequences detected; and wherein at least one of the variant sequences is in at least a portion of CYLD.

In **a second aspect,** the invention pertains a method for diagnosing the presence or an increased risk of developing an autism spectrum disorder, or intellectual disability (ID), in a subject, the method comprising: obtaining a biological sample from a tissue or body fluid sample obtained from a subject; conducting an assay to identify whether in the biological sample there is (i) reduced expression of a CYLD gene, or (ii) reduced activity and/or stability of a CYLD protein; and wherein such reduced expression in (i) and/or reduced activity and/or stability in (ii) is indicative for the presence or an increased risk of developing an autism spectrum disorder in the subject.

In **a third aspect,** the invention pertains to a method for identifying mutations correlated with the presence or increased risk of developing an autism spectrum disorder, the method comprising: identifying a nucleic acid to be evaluated as having a sequence that if mutated may be or is associated with the development of autism; obtaining a nucleic acid sample from a tissue or body fluid sample obtained from a subject having an autism spectrum disorder, or ID; and conducting an assay to identify whether there is a mutation in the nucleic acid sequence in the subject having autism as compared to the nucleic acid sequence in individuals who do not have an autism spectrum disorder, or ID, wherein the presence of the mutation in a subject with an autism spectrum disorder, or ID, indicates that the mutation may be associated with the development of the autism spectrum disorder, or ID, wherein the nucleic acid sequence for which the presence or absence if a mutation is evaluated is at least a portion of the CYLD gene.

In **a fourth aspect,** the invention pertains to a use of an isolated nucleic acid in the method of any one of claims 1 to 7, wherein the isolated nucleic acid comprises a sequence or a variant sequence from a CYLD gene (preferably human CYLD), wherein the variant sequence comprises a genetic variant that is indicative of an autism spectrum disorder, or ID.

In **a fifth aspect,** the invention pertains to a method for screening compounds or compositions for a modulator of a neuropsychiatric manifestation associated with an autism spectrum disorder, or ID, comprising the steps of:
- Bringing into contact a candidate compound or composition with (i) a CYLD protein and/or (ii) a CYLD nucleic acid;
- Determining in (i) an activity and/or stability of the CYLD protein in presence and in absence of the candidate compound or composition; and/or determining in (II) a protein- or mRNA-expression from the CYLD nucleic acid, or a stability of the CYLD nucleic acid, in presence and in absence of the candidate compound or composition;

Wherein, as determined in (i), an increased or reduced activity and/or stability of the CYLD protein in presence compared to absence of the candidate compound or composition, and/or wherein, as determined in (ii), an increased or reduced protein- or mRNA-expression from the CYLD nucleic acid, or an increased or reduced stability of the CYLD nucleic acid, in presence compared to absence of the candidate compound or composition, indicates the candidate compound or composition as a modulator of a neuropsychiatric manifestation associated with an autism spectrum disorder, or ID.

In **a sixth aspect,** the invention pertains to an *in-vivo* method for screening compounds or compositions for a modulator of a neuropsychiatric manifestation associated with an autism spectrum disorder, or ID, comprising the steps of:
- Administering to a non-human animal a candidate compound or composition;
- Determining (quantifying) in the non-human animal at least one neuropsychiatric manifestation associated with an autism spectrum disorder, or ID, compared to a non-human animal that did not receive the candidate compound or composition;

Wherein, as determined in (b), an increased or reduced neuropsychiatric manifestation associated with an autism spectrum disorder, or ID, in the non-human animal that received the candidate compound or composition compared to the non-human animal that did not receive the candidate compound or composition indicates the candidate compound or composition as modulator of a neuropsychiatric manifestation associated with an autism spectrum disorder, or ID.

In **a seventh aspect,** the invention pertains to a compound or composition for use in the treatment of an autism spectrum disorder, or ID, in a subject, wherein the compound or composition is a CYLD protein or a variant or fragment thereof, or is a CYLD nucleic acid suitable for the expression of the CYLD protein or variant or fragment thereof.

In **an eighth aspect,** the invention pertains to a genetically modified non-human animal, preferably a mouse or rat, wherein the transgenic non-human animal comprises at least one genetic mutation within the endogenous *CYLD* locus, and / or at least one recombinant genetic construct that modulates expression, function and / or stability of a CYLD protein, preferably within a central nervous system of the non-human animal.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a method for diagnosing the presence or an increased risk of developing an autism spectrum disorder, or ID, in a subject, the method comprising: obtaining a nucleic acid from a tissue or body fluid sample obtained from a subject; conducting an assay to identify whether there is a variant sequence, or a plurality of variant sequences, in the subject's nucleic acid; for each variant detected, determining if the variant is a known variant associated with an autism spectrum disorder, or ID, or a previously undescribed variant; if the variant is a previously undescribed variant, determining if the variant is expected to have a deleterious effect on at least one of gene expression and/or protein function; and diagnosing the presence or an increased risk of developing the autism spectrum disorder, or ID, based on the variant sequence or the plurality of variant sequences detected; and wherein at least one of the variant sequences is in at least a portion of CYLD.

In **a second aspect,** the invention pertains a method for diagnosing the presence or an increased risk of developing an autism spectrum disorder, or ID, in a subject, the method comprising: obtaining a biological sample from a tissue or body fluid sample obtained from a subject; conducting an assay to identify whether in the biological sample there is (i) reduced expression of a CYLD gene, or (ii) reduced activity and/or stability of a CYLD protein; and wherein such reduced expression in (i) and/or reduced activity and/or stability in (ii) is indicative for the presence or an increased risk of developing an autism spectrum disorder, or ID, in the subject.

In **a third aspect,** the invention pertains to a method for identifying mutations correlated with the presence or increased risk of developing an autism spectrum disorder, or ID, the method comprising: identifying a nucleic acid to be evaluated as having a sequence that if mutated may be or is associated with the development of autism; obtaining a nucleic acid sample from a tissue or body fluid sample obtained from a subject having an autism spectrum disorder or ID,; and conducting an assay to identify whether there is a mutation in the nucleic acid sequence in the subject having autism as compared to the nucleic acid sequence in individuals who do not have an autism spectrum disorder, or ID, wherein the presence of the mutation in a subject with an autism spectrum disorder, or ID, indicates that the mutation may be associated with the development of the autism spectrum disorder, or ID, wherein the nucleic acid sequence for which the presence or absence if a mutation is evaluated is at least a portion of the CYLD gene.

Ubiquitin carboxyl-terminal hydrolase (CYLD) is a deubiquitinase that specifically cleaves 'Lys-63'- and linear 'Met-1'-linked polyubiquitin chains and is involved in NF-kappa-B activation and TNF-alpha-induced necroptosis. The amino acid sequence of CYLD isoforms in humans are provided herein below, and can be derived from the UniProt database under accession No. Q9NQC7 (www.uniprot.org; in the version of Aug-27-2021). CYLD has three cytoskeletal-associated protein-glycine-conserved (CAP-GLY) domains that functions as a deubiquitinating enzyme. Mutations in the CYLD gene have been associated with cylindromatosis, multiple familial trichoepithelioma, and Brooke-Spiegler syndrome. Alternate transcriptional splice variants, encoding different isoforms, have been characterized. The CYLD gene is located in humans on Chr 16: 50.74 - 50.8, and can be accessed in the human genome database (www.gennames.org) with the accession number HGNC:2584 (or NCBI identifier Gene ID: 1540, in the version as updated on 22-Aug-2021; see https://www.ncbi.nlm.nih.gov/gene/1540). Sequences of the respective proteins of human and mouse are provided in SEQ ID NO: 1-5.

In some embodiments, the method may comprise obtaining a nucleic acid from a tissue or body fluid sample from a subject and sequencing at least a portion of a nucleic acid in order to obtain a sample nucleic acid sequence for at least one gene. In certain embodiments, the method may comprise comparing the variant to known variants associated with an autism spectrum disorder, or ID, and determining whether the variant is a variant that has been previously identified as being associated with autism. Or, the method may comprise identifying the variant as a new, previously uncharacterized variant. If the variant is a new variant, or in some cases for previously characterized (i.e., identified) variants, the method may further comprise performing an analysis to determine whether the mutation is expected to be deleterious to expression of the gene and/or the function of the protein encoded by the gene. The method may further comprise using the variant profile (i.e., a compilation of variants identified in the subject) to diagnose the presence of an autism spectrum disorder or an increased risk of developing an autism spectrum disorder.

In embodiments of each of the methods of the invention, the method may comprise performing the assay (e.g., sequencing) in a plurality of individuals to determine the statistical significance of the association.

In various embodiments of the methods of the invention and as described in more detail herein, the assay comprises at least one of nucleic acid sequencing (such as next generation sequencing), hybrid capture, and/or epigenetic analysis. For example, in certain embodiments, next generation (massively-parallel sequencing) may be used. Or, Sanger sequencing may be used. Or, a combination of next generation (massively-parallel sequencing) and Sanger sequencing may be used. Additionally and/or alternatively, the sequencing comprises at least one of single-molecule sequencing-by-synthesis. Thus, in certain embodiments, a plurality of DNA samples are analyzed in a pool to identify samples that show a variation. Additionally or alternatively, in certain embodiments, a plurality of DNA samples are analyzed in a plurality of pools to identify an individual sample that shows the same variation in at least two pools.

Also, in various embodiments, the nucleic acid in the conducting step comprises a gene, an RNA, an exon, an intron, a gene regulatory element, an expressed RNA, an siRNA, or an epigenetic element. Also, regulatory elements, including splice sites, transcription factor binding, A-I editing sites, microRNA binding sites, and functional RNA structure sites may be evaluated for mutations (i.e., variants).

In certain embodiments, the nucleic acid selected for analyzing for a variant comprises a sequence selected from a sequence known or suspected to be associated with one or more autism spectrum disorders. For, example, the nucleic acid comprises at least a portion of one of the gene of CYLD, or any mRNA expressed by that gene. Or, the nucleic acid may comprise a gene that encodes for a protein such as CYLD or a variant protein, homolog, paralog or orthologue thereof.

A biological sample in context of the invention may be any sample of a subject containing any biological material such as genetic material or proteinaceous material. Such samples may be liquid samples or tissue samples. Preferably, the sample of is obtained from a central nervous system of the subject, and preferably is a brain sample such as a brain tissue sample or cerebrospinal fluid sample. In general, the techniques for sequencing nucleic acids (both DNA and RNA) are highly sensitive and therefore, can be used almost any biological sample (i.e., tissue or body fluid) taken from subject. For example, in alternate embodiments, the body fluid comprises at least one of cerebrospinal fluid, blood, amniotic fluid, maternal blood, or urine.

In the various embodiments of the methods of the invention, the autism spectrum disorder may be at least one of non-syndromic autism, classical autism, Asperger's syndrome, Rett's syndrome, childhood disintegrative disorder, or pervasive developmental disorder not otherwise specified (PDD-NOS). In certain embodiments, the autism spectrum disorder comprises non-syndromic autism (i.e., patients who display symptoms of autism but who do not exhibit physical manifestations often found with autism).

Where the present invention pertains to prevention and/or treatment, or a diagnosis of, an autism spectrum disorder, the respective embodiment or aspect is similarly effective in the treatment of a condition referred to as intellectual disability (ID) or mental retardation (both referred to herein as ID). The term "intellectual disability" or "ID" therefore refers to a disability originating at young age, such as an ager before 18, characterized by significant limitations in both intellectual functioning and adaptive behaviour as expressed in conceptual, social, and practical adaptive skills. Such disorder comprise the ability to understand new or complex information, to learn new skills, with a reduced ability to cope independently, which started before adulthood, with a lasting effect on development.

In certain preferred embodiments it might be advantageous that the autism spectrum disorder is increased and as such indicates an involvement in disorders and conditions showing opposite phenotypes of typical autism spectrum disorders. Such may be a general impaired ability of concentration, such as hyperactivity disorder, attention deficit hyperactivity disorder (ADHD) and similar conditions.

The methods of the invention may further comprise diagnosing a presence of, or an increased risk of developing, a genetic syndrome linked to autism, wherein the genetic syndrome comprises a manifesting phenotype. For example, in certain embodiments, the genetic syndrome comprises at least one of Phelan-McDermid/22q13.3 deletion syndrome, Angelman syndrome, Prader-Willi syndrome, 15q11-q13 duplication, fragile X syndrome, fragile X premutation, deletion of chromosome 2q, XYY syndrome, Smith-Lemli-Opitz syndrome, Apert syndrome, mutations in the ARX gene, De Lange syndrome, Smith-Magenis syndrome, Williams syndrome, Noonan syndrome, Down syndrome, velo-cardio-facial syndrome, myotonic dystrophy, Steinert disease, tuberous sclerosis, Duchenne's disease, Timothy syndrome, 10p terminal deletion, Cowden syndrome, 45,X/46,XY mosaicism, Myhre syndrome, Sotos syndrome, Cohen syndrome, Goldenhar syndrome, Joubert syndrome, Lujan-Fryns syndrome, Moebius syndrome, hypomelanosis of Ito, neurofibromatosis type 1, CHARGE syndrome, and/or HEADD syndrome.

The methods may be used to assist in the diagnosis of individuals who do not yet display symptoms of an ASD, or for whom, the diagnosis is equivocal. For example, the subject may be a child or a fetus.

Thus, the method may comprise a method for identifying mutations correlated with the presence or increased risk of developing an autism spectrum disorder, comprising: identifying a CYLD nucleic acid to be evaluated as having a sequence that if mutated may be or is associated with the development of autism; obtaining a nucleic acid sample from a tissue or body fluid sample from a subject having an autism spectrum disorder; and conducting an assay to identify whether there is a mutation in the CYLD nucleic acid sequence in the subject having autism as compared to the CYLD nucleic acid sequence in individuals who do not have an autism spectrum disorder, wherein the presence of the mutation in a subject with an autism spectrum disorder indicates that the mutation may be associated with the development of the autism spectrum disorder.

In embodiments the methods of the invention for identifying new mutations, the method may comprise performing the assay (e.g., sequencing) in a plurality of individuals to determine the statistical significance of the association.

In the various embodiments of the methods of the invention, the autism spectrum disorder may be at least one of non-syndromic autism, classical autism, Asperger's syndrome, Rett's syndrome, childhood disintegrative disorder, or pervasive developmental disorder not otherwise specified (PDD-NOS). In certain embodiments, the autism spectrum disorder comprises non-syndromic autism. Or, the association of variants with other syndromes that are associated (e.g., genetically linked to) with autism, such as at least one of Phelan-McDermid/22q13.3 deletion syndrome, Angelman syndrome, Prader-Willi syndrome, 15q11-q13 duplication, fragile X syndrome, fragile X premutation, deletion of chromosome 2q, XYY syndrome, Smith-Lemli-Opitz syndrome, Apert syndrome, mutations in the ARX gene, De Lange syndrome, Smith-Magenis syndrome, Williams syndrome, Noonan syndrome, Down syndrome, velo-cardio-facial syndrome, myotonic dystrophy, Steinert disease, tuberous sclerosis, Duchenne's disease, Timothy syndrome, 10p terminal deletion, Cowden syndrome, 45,X/46,XY mosaicism, Myhre syndrome, Sotos syndrome, Cohen syndrome, Goldenhar syndrome, Joubert syndrome, Lujan-Fryns syndrome, Moebius syndrome, hypomelanosis of Ito, neurofibromatosis type 1, CHARGE syndrome, and/or HEADD syndrome.

In various embodiments, of the methods of the invention and as described in more detail herein, the assay comprises at least one of nucleic acid sequencing, hybrid capture, and epigenetic analysis. For example, in certain embodiments, next generation (massively-parallel sequencing) may be used. Or, Sanger sequencing may be used. Or, a combination of next generation (massively-parallel sequencing) and Sanger sequencing may be used. Additionally and/or alternatively, the sequencing comprises at least one of single-molecule sequencing-by-synthesis. Thus, in certain embodiments, a plurality of DNA samples are analyzed in a pool to identify samples that show a variation. Additionally or alternatively, in certain embodiments, a plurality of DNA samples are analyzed in a plurality of pools to identify an individual sample that shows the same variation in at least two pools.

Also, in various embodiments, the nucleic acid in the conducting step comprises a CYLD gene, a CYLD RNA, a CYLD exon, a CYLD intron, a CYLD gene regulatory element, a CYLD expressed RNA, a CYLD siRNA, or a CYLD epigenetic element. Also, regulatory elements, including splice sites, transcription factor binding, A-I editing sites, microRNA binding sites, and functional RNA structure sites of CYLD or in the proximity (+/- about 10kb, preferably about 5kb) of the CYLD gene may be evaluated for mutations (i.e., variants).

The methods may be used to assist in the diagnosis of individuals who do not yet display symptoms of an ASD, or for whom, the diagnosis is equivocal. For example, the subject may be a child or a fetus.

In preferred embodiments of the invention the method comprises a determining if the mutation is expected to have a deleterious effect on at least one of gene expression and/or protein function.

In context of the invention the term "mutation" or "variant" shall refer to a change in nucleic acid sequence, or amino acid sequence, compared to the herein disclosed sequences of CYLD nucleic acids or CYLD proteins. As such preferred mutations or variants usable as biomarkers in accordance with the invention are "single nucleotide polymorphism" or "SNP". The term refers to variation in a single nucleotide that occurs at a specific position in the genome, where each variation is present to some appreciable degree within a population (e.g. >1%). For most SNPs so far discovered, there are only two different bases that occur at that position, but SNPs with three different base variations have been described. SNPs underlie differences in susceptibility to disease; a wide range of human diseases, e.g. sickle-cell anemia, β-thalassemia and cystic fibrosis result from SNPs. The severity of illness and the responsiveness to treatments can also depend on genetic variations. For example, in humans, a single base mutation in the APOE (apolipoprotein E) gene is associated with a higher risk for Alzheimer's disease. The term "variant" refers to any alteration in a nucleic acid that changes its nucleotide sequence. Hence, CYLD SNPs are candidate variants as biomarkers to detect an association with autism spectrum disorders. Therefore, and preferably, the methods of the invention include determining if the mutation has an effect of on enzymatic activity of CYLD protein to remove Lysine 63 (K63)-linked polyubiquitin chain from a substrate protein.

It is generally preferred that in context of the present invention the CYLD gene and/or CYLD protein is/are mammalian, preferably human.

In **a fourth aspect,** the invention pertains to a use of an isolated nucleic acid in the method of any one of the above referenced aspects and embodiments, wherein the isolated nucleic acid comprises a sequence or a variant sequence from a CYLD gene (preferably human CYLD), wherein the variant sequence comprises a genetic variant that is indicative of an autism spectrum disorder. Such variant as described above, preferably, is a mutation or a SNP.

In a **fifth aspect,** the invention pertains to a method for screening compounds or compositions for a modulator of a neuropsychiatric manifestation associated with an autism spectrum disorder, comprising the steps of:
- Bringing into contact a candidate compound or composition with (i) a CYLD protein and/or (ii) a CYLD nucleic acid;
- Determining in (i) an activity and/or stability of the CYLD protein in presence and in absence of the candidate compound or composition; and/or determining in (II) a protein- or mRNA-expression from the CYLD nucleic acid, or a stability of the CYLD nucleic acid, in presence and in absence of the candidate compound or composition;
Wherein, as determined in (i), an increased or reduced activity and/or stability of the CYLD protein in presence compared to absence of the candidate compound or composition, and/or wherein, as determined in (ii), an increased or reduced protein- or mRNA-expression from the CYLD nucleic acid, or an increased or reduced stability of the CYLD nucleic acid, in presence compared to absence of the candidate compound or composition, indicates the candidate compound or composition as a modulator of a neuropsychiatric manifestation associated with an autism spectrum disorder.

The reduction (or enhancement) of expression, activity function and/or stability or CYLD is, preferably, identified by reference to a control method. In one example, the control method may be one practiced in the absence of any candidate compound, or with a compound having a known effect on such expression, function, activity and/or stability (such as a positive or negative control), of CYLD.

In certain embodiments of the screening of the invention, the CYLD protein is provided in context of a biological cell, such as via expressing a CYLD protein in a biological cell and contacting the CYLD protein with the candidate compound by introducing the candidate compound into the cell expressing the CYLD protein. In such embodiments the CYLD protein may be endogenously expressed in said cell, or may be expressed using one or more nucleic acid constructs comprising a sequence encoding the CYLD protein which are operatively linked to one or more expression elements (promoter, enhancer etc.). The CYLD protein so expressed may be a wild-type sequence of for example human CYLD, or any alternative variant CYLD protein, such as an enzymatically inactive or hyperactive CYLD protein.

The candidate compound used in the screening methods may be one selected from a polypeptide, peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, or small cell-permeable molecules. Small molecules are in certain embodiments molecules with a molecular weight of less than 5kD, or preferably less than 1kD.

In a **sixth aspect,** the invention pertains to an *in-vivo* method for screening compounds or compositions for a modulator of a neuropsychiatric manifestation associated with an autism spectrum disorder, comprising the steps of:
- Administering to a non-human animal a candidate compound or composition;
- Determining (quantifying) in the non-human animal at least one neuropsychiatric manifestation associated with an autism spectrum disorder compared to a non-human animal that did not receive the candidate compound or composition;

Wherein, as determined in (b), an increased or reduced neuropsychiatric manifestation associated with an autism spectrum disorder in the non-human animal that received the candidate compound or composition compared to the non-human animal that did not receive the candidate compound or composition indicates the candidate compound or composition as modulator of a neuropsychiatric manifestation associated with an autism spectrum disorder.

Preferred in this context is that the non-human animal is a mammal, preferably a mouse, a rat, a rabbit, or a monkey. Preferably, the non-human animal is characterized by a reduced expression, function and/or stability of CYLD protein, such as a CYLD genetic knock-out or knock-down (RNAi) animal, and wherein the modulator to be screened is an antagonist of a neuropsychiatric manifestation associated with an autism spectrum disorder, such a disorder as defined elsewhere herein.

As such it should be understood that preferably the modulator of a neuropsychiatric manifestation associated with an autism spectrum disorder is agonist or an antagonist of a neuropsychiatric manifestation associated with an autism spectrum disorder.

However, the present invention may also include embodiments for screening agonists of CYLD as compounds which are advantageous in a clinical scenario where the autism spectrum disorder phenotype should be increased, and as such indicates a therapeutic applicability in disorders and conditions showing opposite phenotypes of typical autism spectrum disorders. Such may be a general impaired ability of concentration, such as hyperactivity disorder, attention deficit hyperactivity disorder (ADHD) and similar conditions.

Such compounds identified in accordance with the method of the invention may be applicable for the treatment of other CYLD related disorders as well, such as Amyotrophic lateral sclerosis (ALS) and frontotemporal lobar degeneration (FTLD).

In **a seventh aspect,** the invention pertains to a compound or composition for use in the treatment of an autism spectrum disorder in a subject, wherein the compound or composition is a CYLD protein or a variant or fragment thereof, or is a CYLD nucleic acid suitable for the expression of the CYLD protein or variant or fragment thereof.

Preferably the treatment involves an administration of the compound or composition to a central nervous system (CNS) of the subject. Such administration therefore is either an administration suitable for a compound or composition to be shuttled across or to pass the blood brain barrier (BBB), or alternatively is directly administered to the CNS. Such administration may include in some embodiments, that the CNS administration is intrathecal (IT) injection, intracerebroventricular (ICV) injection, or intracisternal injection. In some embodiments, a modified lysosomal enzyme or pharmaceutical composition as described herein is administered intraparenchymally by direct injection. The route of administration preferably is suitable to allow and involve an administration to a postsynaptic neuron in the CNS of the subject.

A treatment in accordance with the present invention in addition may comprise a gene therapeutic introduction of a CYLD nucleic acid, or CYLD variant nucleic acid into the subject. The term "gene therapy" generally means a method of therapy wherein a desired gene/genetic sequence is inserted into a cell or tissue (along with other sequences necessary for the expression of the specific gene). See, for example, genetherapynet.com for description of gene therapy techniques.

The term "subject" as used herein can include a human subject for medical purposes, such as for the treatment of an existing disease, disorder, condition or the prophylactic treatment for preventing the onset of a disease, disorder, or condition or an animal subject for medical, veterinary purposes, or developmental purposes. Suitable animal subjects include mammals including, but not limited to, primates, e.g., humans, monkeys, apes, gibbons, chimpanzees, orangutans, macaques and the like; bovines, e.g., cattle, oxen, and the like; ovines, e.g., sheep and the like; caprines, e.g., goats and the like; porcines, e.g., pigs, hogs, and the like; equines, e.g., horses, donkeys, zebras, and the like; felines, including wild and domestic cats; canines, including dogs; lagomorphs, including rabbits, hares, and the like; and rodents, including mice, rats, guinea pigs, and the like. An animal may be a transgenic animal. In some embodiments, the subject is a human including, but not limited to, fetal, neonatal, infant, juvenile, and adult subjects. Further, a "subject" can include a patient afflicted with or suspected of being afflicted with a disease, disorder, or condition. Thus, the terms "subject" and "patient" are used interchangeably herein. Subjects also include animal disease models (e.g., rats or mice used in experiments, and the like).

Gene therapy delivery constructs containing an isolated DNA or mRNA encoding a CYLD protein (SEQ. ID. No. 1 to 3) are preferably delivered via any known gene therapy shuttle and may therefore be delivered via an adenoviral associated virus (AAV). There are further disclosed pharmaceutical compositions and methods for treating, preventing or reducing symptoms of the diseases and conditions mentioned.

Preferably, the compounds of the invention are provided in this context as a pharmaceutical composition wherein the active ingredient (the compound) is formulated together with a pharmaceutically acceptable carrier and/or excipient.

In **an eighth aspect,** the invention pertains to a genetically modified non-human animal, preferably a mouse or rat, wherein the transgenic non-human animal comprises at least one genetic mutation within the endogenous *CYLD* locus, and / or at least one recombinant genetic construct that modulates expression, function and / or stability of a CYLD protein, preferably within a central nervous system of the non-human animal.

Preferably the genetically modified non-human animal of the invention has an increased or decreased expression, function and/or activity of a CYLD protein compared to a non-human animal not comprising said at least one genetic mutation and said at least one genetic construct.

A genetically modified non-human animal of the invention is preferred, wherein the genetic modification is a loss of function or gain of function mutation of the CYLD gene.

A CYLD protein or gene is this context can either be the endogenously expressed CYLD, such as if the non-human animal is a mouse, the CYLD protein or gene is murine; or alternatively a heterologous modification can be introduced into the non-human animal, such that for example a human CYLD is expressed in a mouse model.

Generally preferred is that the genetic modification is a knock-out of the CYLD gene, and/or wherein the at least one genetic construct expresses an RNA interference construct for use as a knock-down of the CYLD gene expression.

In certain embodiments it may be preferred that the genetic modification and/or the genetic construct is a conditional construct and present or inducible only in a cell or tissue of the CNS.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

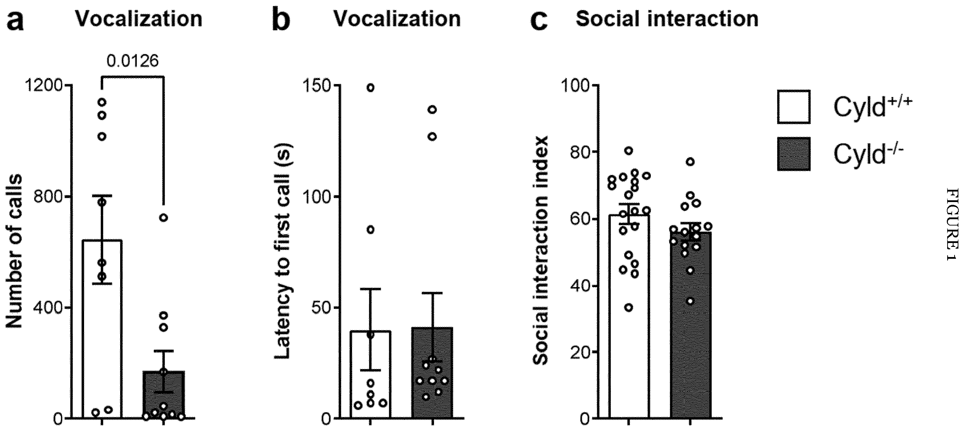

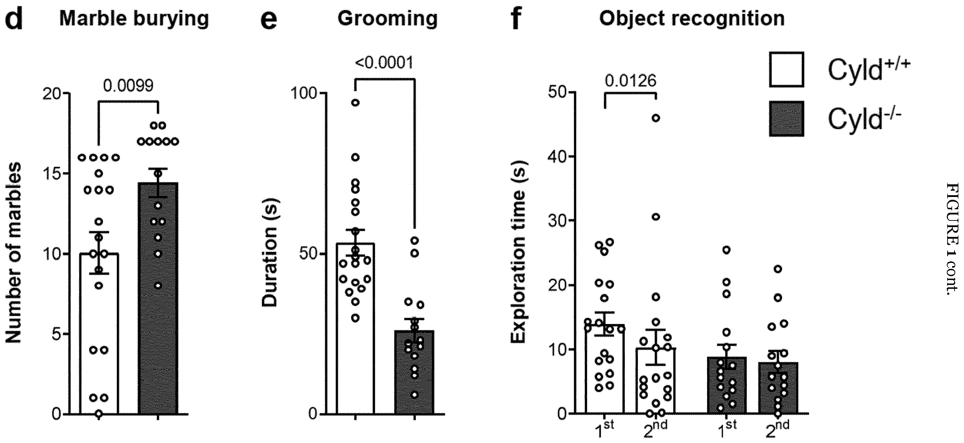

**Figure 1****:** *Cyld*^{*-*/*-*} mice display autism-like phenotypes including an impairment of social communication, increased repetitive behavior and cognitive dysfunction, **a-b** Vocalization analysis of number of calls emitted in the presence of a female mouse, and latency to first call. **c** Social index of social interaction analysis in the three-chamber test. **d-e** Repetitive behavior assessment with both marble burying test and time of grooming. **f** Analysis of the exploratory behavior with time of the first and the second exposure to the same object. For experiments in a-b, n = 8 for *Cyld*^{+/+} controls and n = 10 for *Cyld*^{-/-}; for experiments in c-f, n = 19 for *Cyld*^{+/+} controls and n = 15 for *Cyld*^{-/-}*.* All n values used for statistics refer to the number of mice used, after identification of possible outliers with Grubbs' method. *P < 0.05, **P < 0.01 and ***P < 0.001, ****P < 0.001 calculated by unpaired nonparametric Mann-Whitney test. Graphs are mean ± s.e.m.

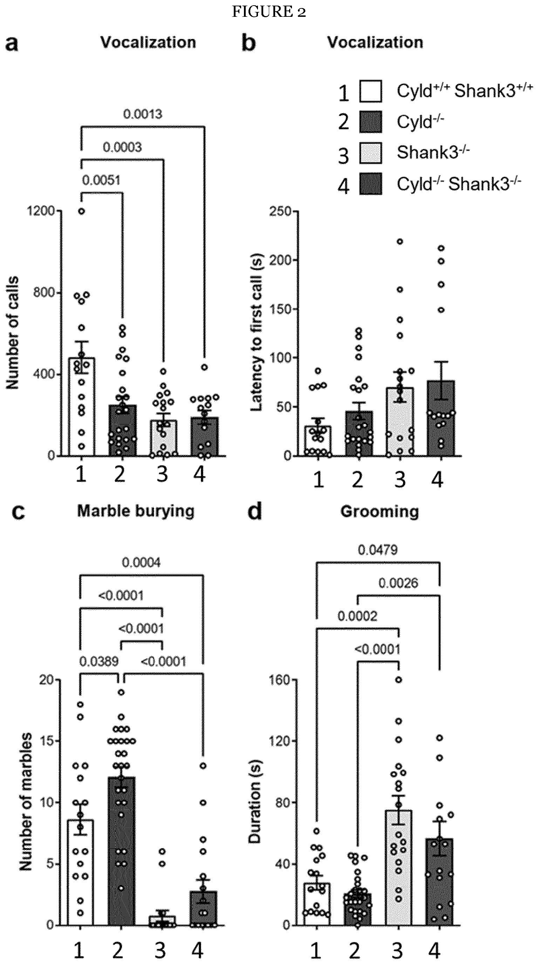

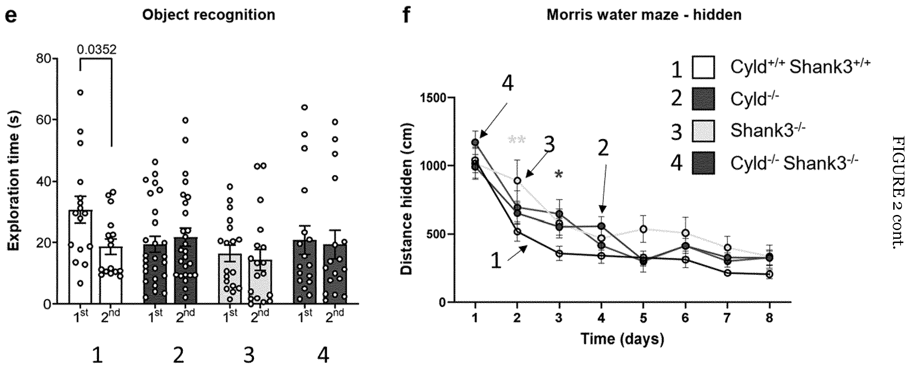

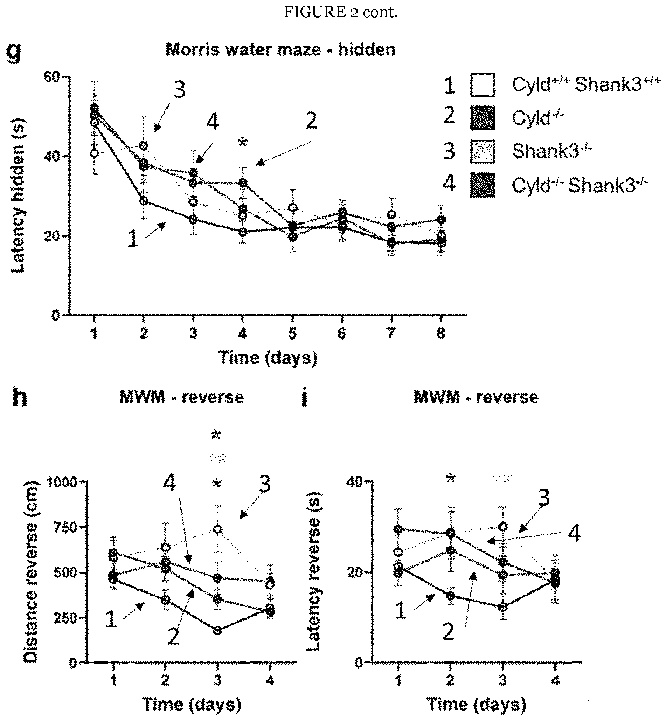

**Figure 2**: Behavioral analysis of *Cyld*^{*-*/}*⁻, Shank3*^{*-*/}*⁻,* and *Cyld*^{*-*/}*⁻Shank3*^{*-*/*-*} double mutant mice. **a-b** Vocalization analysis of number of calls emitted in the presence of a female mouse, and latency to first call. **c-d** Repetitive behavior assessment with both marble burying test and time of grooming. **e** Analysis of the exploratory behavior with time of the first and the second exposure to the same object. **f-i** Analysis of spatial memory by Morris-Water Maze test, with measures of total distance travelled and latency to the platform during hidden training phase, and reverse phase. For experiments in a-b, n = 15 for *Cyld*^{+/+}*Shank3*^{+/+} controls, n = 22 for *Cyld*^{*-*/*-*} mice, n = 17 for *Shank3*^{*-*/}*⁻,* and n = 14 for *Cyld*^{*-*/}*⁻Shank3*^{*-*/*-*} mice, from 4 independent cohorts. For experiments in c-e, n = 16 for *Cyld*^{+/+}*Shank3*^{+/+} controls, n = 26 for *Cyld*^{*-*/*-*} mice, n = 18 for *Shank3*^{*-* /-}, and n = 17 for *Cyld*^{*-*/}*⁻Shank3*^{*-*/*-*} mice, from 4 independent cohorts. For experiments in f-i, n = 15 for *Cyld*^{+/+}*Shank3*^{+/+} controls, n = 22 for *Cyld*^{*-*/*-*} mice, n = 17 for *Shank3*^{*-*/}*⁻,* and n = 13 for *Cyld*^{*-*/}*⁻ Shank3*^{*-*/*-*} mice, from 4 independent cohorts. All n values used for statistics refer to the number of mice used, after identification of possible outliers with Grubbs' method. *P < 0.05, **P < 0.01, ***P < 0.001, ****P < 0.001 calculated by ordinary one-way ANOVA followed by Tukey's multiple comparisons test (a-d), by unpaired nonparametric Mann-Whitney test (e), or by two-way repeated-measures analysis of variance (ANOVA) with Dunnett's multiple comparisons test (f-h). Graphs are mean ± s.e.m.

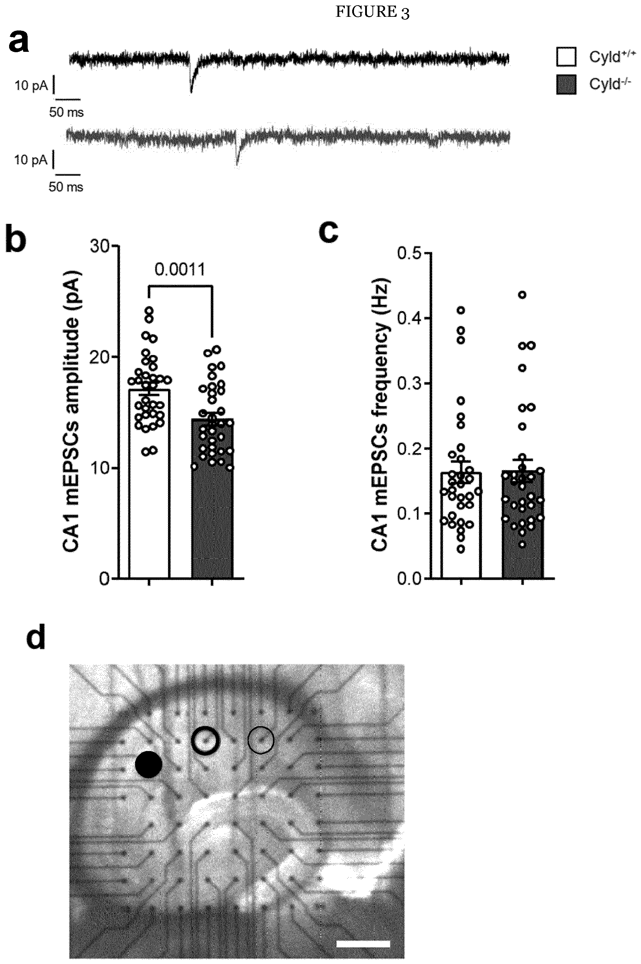

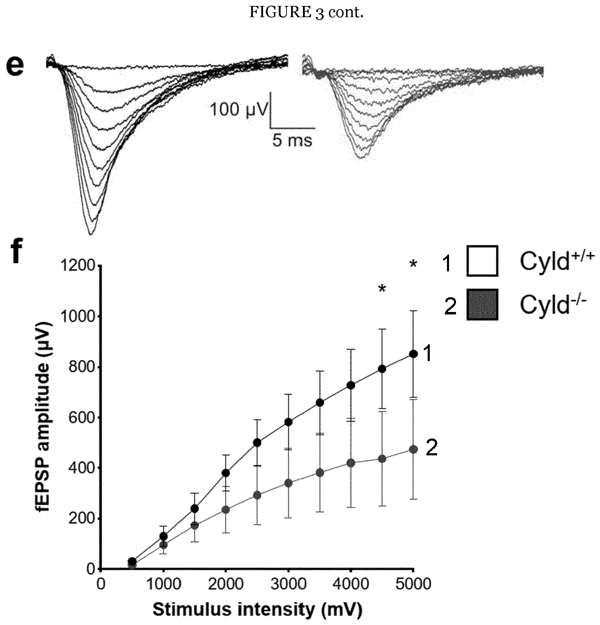

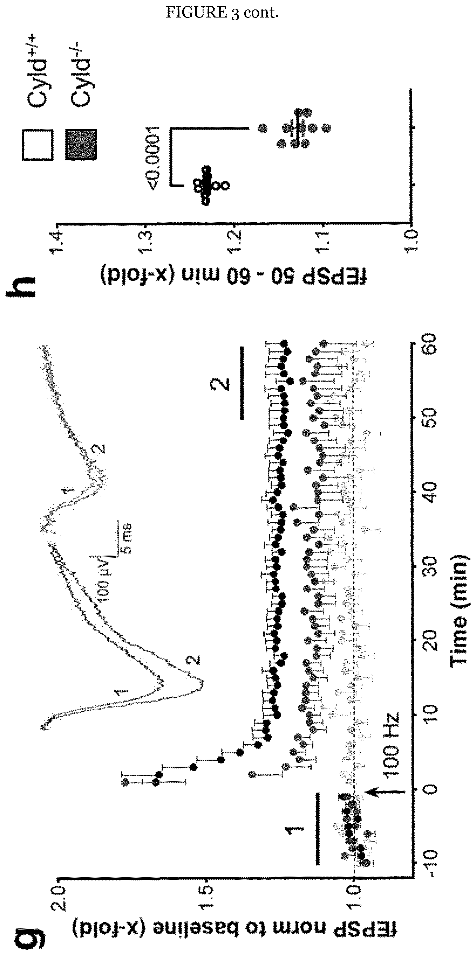

**Figure 3****:** Electrophysiological analysis of CA1 mEPSCs and fEPSPs highlighted an impaired excitability in *Cyld*^{*-*/*-*} mice. **a-c** Representative traces of AMPA-mediated mEPSCs and quantification of mEPSC frequency and amplitude in the hippocampal CA1 region of *Cyld*^{*-*/*-*} mice and *Cyld*^{+/+} controls at P42. **d** Representative picture of the location of an acute hippocampal slice on top of the MEA chip. The red filled dot indicates the stimulation electrode, the violet circle marks the recording electrode and the empty red circle marks the 2nd. stimulation electrode of the independent control pathway. **e** Representative traces of fEPSP at gradually increasing stimulus intensity generate input output (I/O) curves in both experimental groups. f The I/O curves show decreased fEPSP amplitudes in *Cyld* ^{*-*/*-*} mice (*P < 0.05). **g** LTP induction of Schaffer collaterals using a 100 Hz HFS. Black dots correspond to the data from *Cyld*^{+/+} (10 slices of 4 mice), violet dot from *Cyld*^{-/-} (11 slices of 4 mice), while the grey dots were recorded from the independent control pathway (11 slices). **h** The relative strength of the potentiation of fEPSPs 50-60 min after HFS was significantly lower in *Cyld*^{-/-} (****P < 0.001). For experiments in a-c, n = 32 neurons from *Cyld*^{+/+} controls and n = 32 neurons from *Cyld*^{-/-} mice. Statistics was calculated by unpaired t test (a-c). Nonparametric Mann-Whitney tests were used to compare two groups displaying a non-normal distribution. 2-way ANOVA was used to compare I/O curve progression and responses to single intensities were compared using Fisher's LSD. *P < 0.05, **P < 0.01, ***P < 0.001, ****P < 0.001 graphs are mean ± s.e.m.

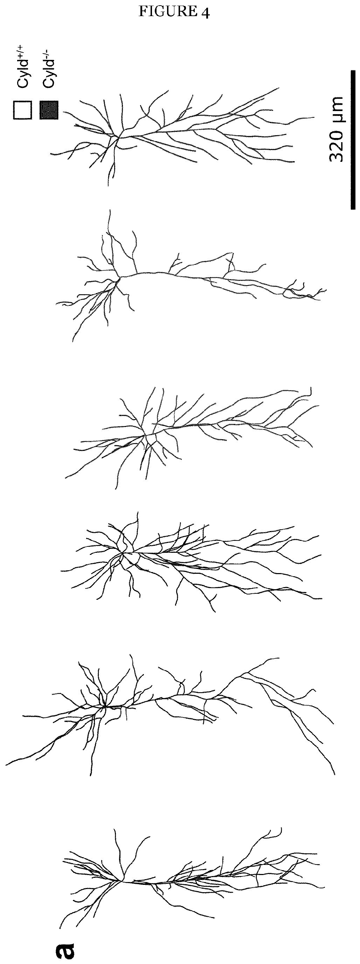

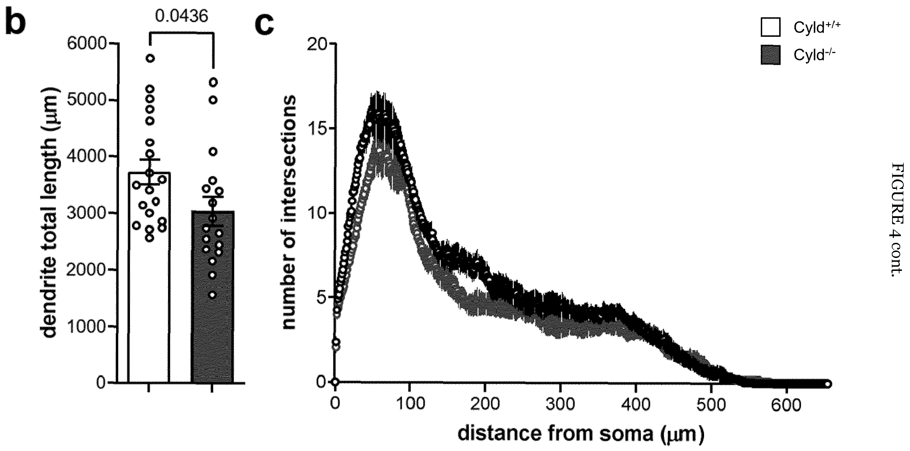

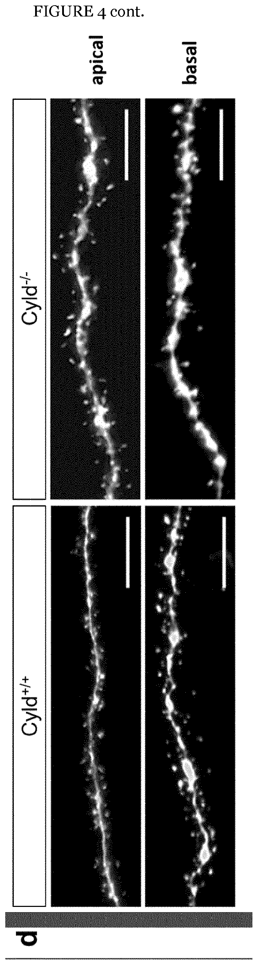

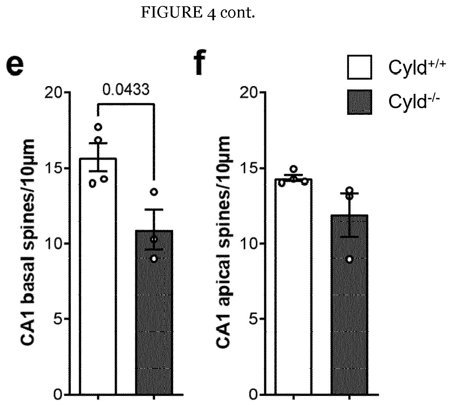

**Figure 4**: CYLD deletion results in reduced total dendrite length and basal spine number of CA1 hippocampal pyramidal neurons (PNs). **a** Representative pictures of reconstructed biocytin filled PNs by IMARIS of *Cyld*^{*-*/*-*} mice and *Cyld*^{+/+} controls at P42 in CA1. **b-c** Measurement of dendrite total length, and Sholl analysis of CA1 PNs. **d** Representative pictures of apical and basal spines of *Cyld*^{*-*/*-*} and *Cyld*^{+/+} PNs. **e-f** Quantification of basal and apical spines. For experiments in a-c, n = 19 neurons from *Cyld*^{+/+} controls and n = 17 neurons from *Cyld*^{*-*/*-*} mice. For experiments in d-f n = 4 for *Cyld*^{+/+} controls and n = 3 for *Cyld*^{*-*/*-*} mice. Statistics was calculated by unpaired t test (b, e-f), and two-way repeated-measures analysis of variance (ANOVA) with Sidak's (c) post-hoc tests. *P < 0.05, graphs are mean ± s.e.m. Error bars in a = 40 µm, error bars in d = 5 µm.

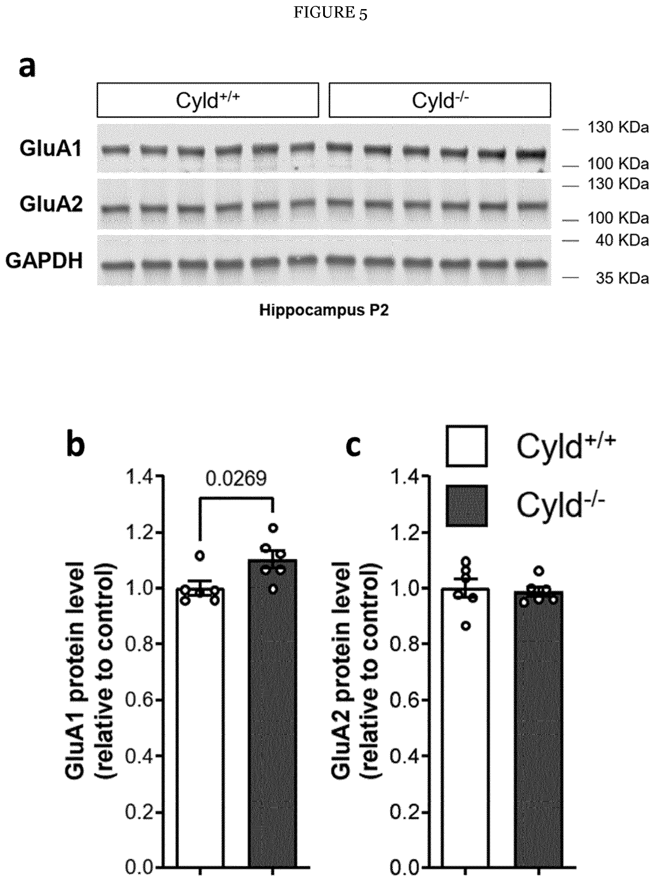

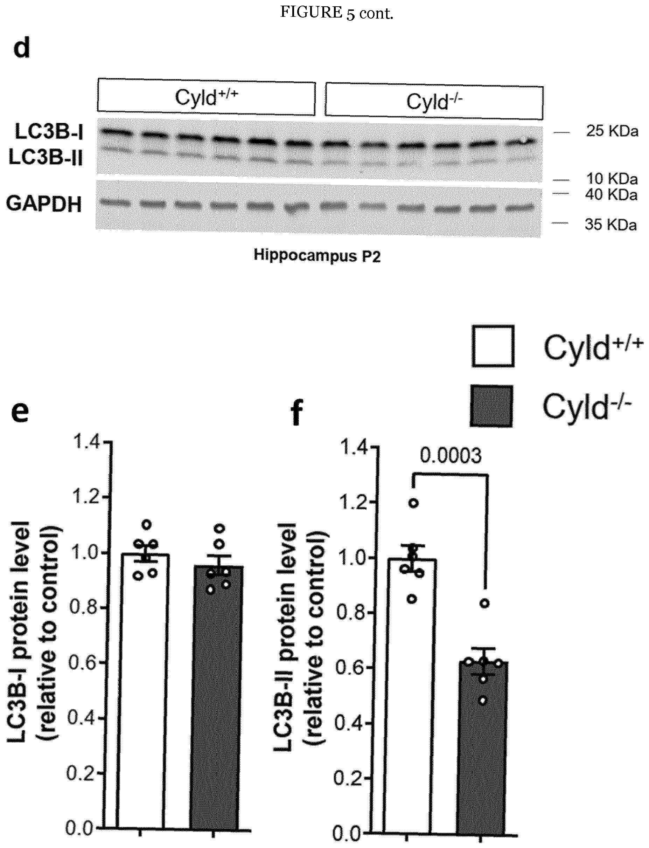

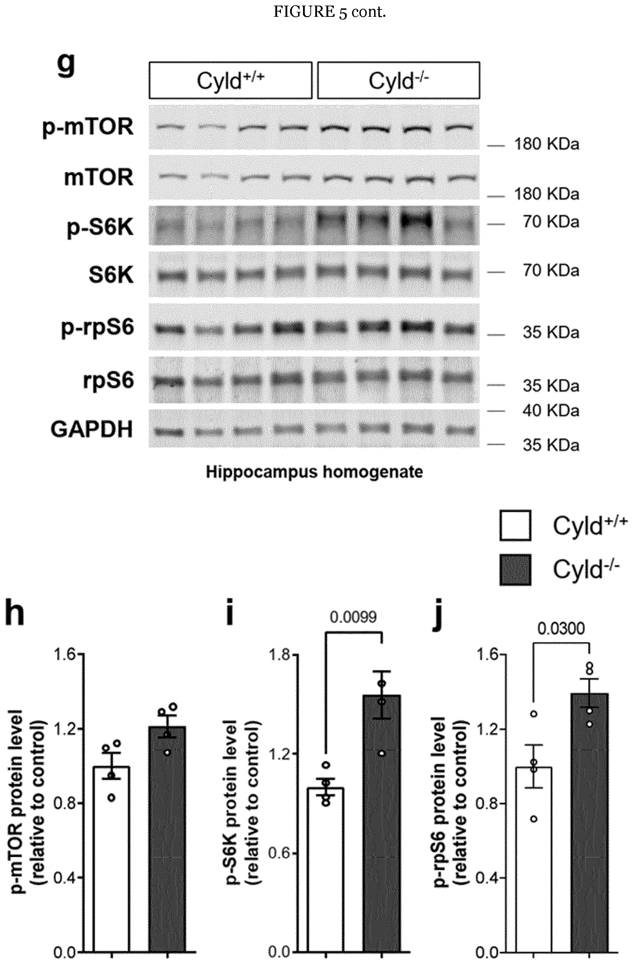

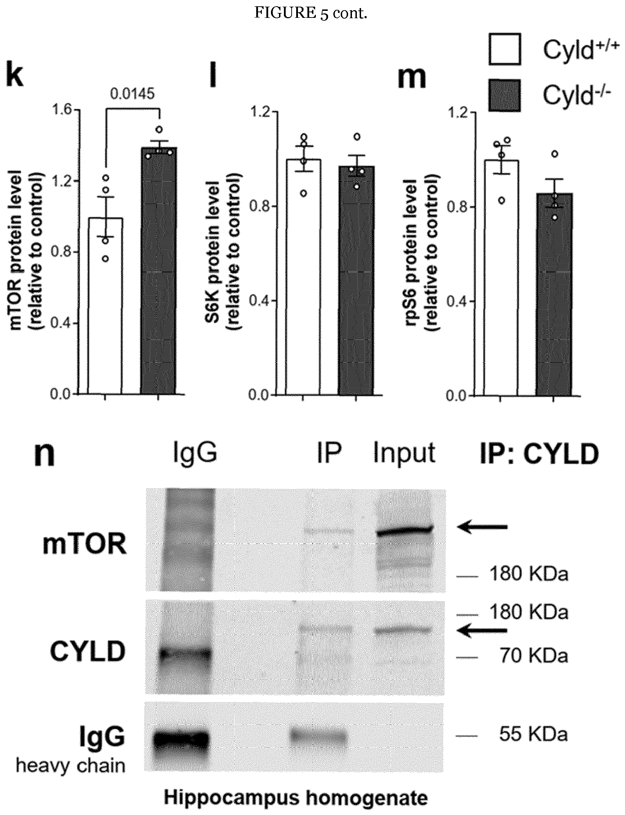

**Figure 5**: The loss of CYLD leads to increase of the AMPA receptor subunit GluA1, dysregulates autophagic flux and causes mTOR signaling upregulation within the hippocampus. **a-c** Western blot analysis of the AMPA receptor subunits GluA1 and GluA2, each normalized to GAPDH in the hippocampal P2 fraction. **d-f** Western blot analysis of LC3B-I and LC3B-II each normalized to GAPDH in the hippocampal P2 fraction. **a-g** Western blot analysis of mTOR signaling cascade and relative quantification in hippocampus homogenate. p-mTOR, p-S6K, p-rpS6 were normalized to total mTOR, S6K, and rpS6 respectively. Total mTOR, total S6K and total rpS6 were normalized to GAPDH. Lysates (20 µg proteins for a, d and 30 µg proteins for g) of *Cyld*^{*-*/*-*} mice and *Cyld*^{+/+} controls were run on a 4-15% gradient gel. **h** CYLD immunoprecipitation from wild-type hippocampus homogenate, run on a 4-15% gradient gel and blotted for mTOR and CYLD. For experiments in a-f, n = 6 for *Cyld*^{+/+} controls and n = 6 for *Cyld*^{*-* /-} mice at P42. For experiments in g-m, n = 4 for *Cyld*^{+/+} controls and n = 4 for *Cyld*^{*-*/*-*} mice at P42. Statistics calculated by unpaired t test, show a significant increase of GluA1, a significant decrease of LC3B-II protein levels, and a significant increase of total mTOR, p-S6K, and

The sequences show:
**SEQ ID NO 1 shows isoform 1 of human CYLD protein**

| |
|---|
| 10 20 30 40 50  160 170 180 190 200 |

| |
|---|
| |

**SEQ ID NO** 2 **shows isoform** 2 **of human CYLD protein**

| |
|---|
|         10         20         30         40         50  |

| |
|---|
| 360 370 380 390 400 LFYTLNGSSV DSQPQSKSKN TWYIDEVAED PAKSLTEIST DFDRSSPPLQ 410 420 430 440 450 PPPVNSLTTE NRFHSLPFSL TKMPNTNGSI GHSPLSLSAQ SVMEELNTAP 460 470 480 490 500 VQESPPLAMP PGNSHGLEVG SLAEVKENPP FYGVIRWIGQ PPGLNEVLAG 510 520 530 540 550 LELEDECAGC TDGTFRGTRY FTCALKKALF VKLKSCRPDS RFASLQPVSN 560 570 580 590 600 QIERCNSLAF GGYLSEVVEE NTPPKMEKEG LEIMIGKKKG IQGHYNSCYL 610 620 630 640 650 DSTLFCLFAF SSVLDTVLLR PKEKNDVEYY SETQELLRTE IVNPLRIYGY 660 670 680 690 700 VCATKIMKLR KILEKVEAAS GFTSEEKDPE EFLNILFHHI LRVEPLLKIR 710 720 730 740 750 SAGQKVQDCY FYQIFMEKNE KVGVPTIQQL LEWSFINSNL KFAEAPSCLI 760 770 780 790 800 IQMPRFGKDF KLFKKIFPSL ELNITDLLED TPRQCRICGG LAMYECRECY 810 820 830 840 850 DDPDISAGKI KQFCKTCNTQ VHLHPKRLNH KYNPVSLPKD LPDWDWRHGC 860 870 880 890 900 IPCQNMELFA VLCIETSHYV AFVKYGKDDS AWLFFDSMAD RDGGQNGFNI 910 920 930 940 950 PQVTPCPEVG EYLKMSLEDL HSLDSRRIQG CARRLLCDAY MCMYQSPTMS LYK |

**SEQ ID NO 3 shows isoform 1 of mouse CYLD protein**

| |
|---|
| 10 20 30 40 50 MSSGLWSQEK VTSPYWEERI FYLLLQECSV TDKQTQKLLK VPKGSIGQYI 60 70 80 90 100 QDRSVGHSRV PSTKGKKNQI GLKILEQPHA VLFVDEKDVV EINEKFTELL 110 120 130 140 150 LAITNCEERL SLFRNRLRLS KGLQVDVGSP VKVQLRSGEE KFPGVVRFRG 160 170 180 190 200 PLLAERTVSG IFFGVELLEE GRGQGFTDGV YQGKQLFQCD EDCGVFVALD 210 220 230 240 250 KLELIEDDDN GLESDFAGPG DTMQVEPPPL EINSRVSLKV GESTESGTVI 260 270 280 290 300 FCDVLPGKES LGYFVGVDMD NPIGNWDGRF DGVQLCSFAS VESTILLHIN 310 320 330 340 350 DIIPALSDSV TQERRPPKLA FMSRGVGDKG SSSHNKPKVT GSTSDPGSRN 360 370 380 390 400 RSELFYTLNG SSVDSQQSKS KNPWYIDEVA EDPAKSLTEM SSDFGHSSPP 410 420 430 440 450 PQPPSMNSLS SENRFHSLPF SLTKMPNTNG SMAHSPLSLS VQSVMGELNS 460 470 480 490 500 TPVQESPPLP ISSGNAHGLE VGSLAEVKEN PPFYGVIRWI GQPPGLSDVL 510 520 530 540 550 AGLELEDECA GCTDGTFRGT RYFTCALKKA LFVKLKSCRP DSRFASLQPV 560 570 580 590 600 SNQIERCNSL AFGGYLSEVV EENTPPKMEK EGLEIMIGKK KGIQGHYNSC 610 620 630 640 650 YLDSTLFCLF AFSSALDTVL LRPKEKNDIE YYSETQELLR TEIVNPLRIY |

| |
|---|
|         660        670        680        690        700 GYVCATKIMK LRKILEKVEA ASGFTSEEKD PEEFLNILFH DILRVEPLLK 710        720        730        740        750 IRSAGQKVQD CNFYQIFMEK NEKVGVPTIQ QLLEWSFINS NLKFAEAPSC 760        770        780        790        800 LIIQMPRFGK DFKLFKKIFP SLELNITDLL EDTPRQCRIC GGLAMYECRE 810        820        830        840        850 CYDDPDISAG KIKQFCKTCS TQVHLHPRRL NHSYHPVSLP KDLPDWDWRH 860        870        880        890        900 GCIPCQKMEL FAVLCIETSH YVAFVKYGKD DSAWLFFDSM ADRDGGQNGF 910        920        930        940        950 NIPQVTPCPE VGEYLKMSLE DLHSLDSRRI QGCARRLLCD AYMCMYQSPT MSLYK |

**SEQ ID NO 4 shows isoform 1 of mouse CYLD protein**

| |
|---|
| 10         20         30         40         50 MSSGLWSQEK VTSPYWEERI FYLLLQECSV TDKQTQKLLK VPKGSIGQYI 60         70         80         90         100 QDRSVGHSRV PSTKGKKNQI GLKILEQPHA VLFVDEKDVV EINEKFTELL 110        120        130        140        150 LAITNCEERL SLFRNRLRLS KGLQVDVGSP VKVQLRSGEE KFPGVVRFRG 160        170        180        190        200 PLLAERTVSG IFFGVELLEE GRGQGFTDGV YQGKQLFQCD EDCGVFVALD 210        220        230        240        250 KLELIEDDDN GLESDFAGPG DTMQVEPPPL EINSRVSLKV GESTESGTVI 260        270        280        290        300 FCDVLPGKES LGYFVGVDMD NPIGNWDGRF DGVQLCSFAS VESTILLHIN 310        320        330        340        350 DIIPDSVTQE RRPPKLAFMS RGVGDKGSSS HNKPKVTGST SDPGSRNRSE 360        370        380        390        400 LFYTLNGSSV DSQQSKSKNP WYIDEVAEDP AKSLTEMSSD FGHSSPPPQP 410        420        430        440        450 PSMNSLSSEN RFHSLPFSLT KMPNTNGSMA HSPLSLSVQS VMGELNSTPV 460        470        480        490        500 QESPPLPISS GNAHGLEVGS LAEVKENPPF YGVIRWIGQP PGLSDVLAGL 510        520        530        540        550 ELEDECAGCT DGTFRGTRYF TCALKKALFV KLKSCRPDSR FASLQPVSNQ 560        570        580        590        600 IERCNSLAFG GYLSEVVEEN TPPKMEKEGL EIMIGKKKGI QGHYNSCYLD 610        620        630        640        650 STLFCLFAFS SALDTVLLRP KEKNDIEYYS ETQELLRTEI VNPLRIYGYV 660        670        680        690        700 CATKIMKLRK ILEKVEAASG FTSEEKDPEE FLNILFHDIL RVEPLLKIRS 710        720        730        740        750 AGQKVQDCNF YQIFMEKNEK VGVPTIQQLL EWSFINSNLK FAEAPSCLII 760        770        780        790        800 QMPRFGKDFK LFKKIFPSLE LNITDLLEDT PRQCRICGGL AMYECRECYD 810        820        830        840        850 DPDISAGKIK QFCKTCSTQV HLHPRRLNHS YHPVSLPKDL PDWDWRHGCI 860        870        880        890        900 PCQKMELFAV LCIETSHYVA FVKYGKDDSA WLFFDSMADR DGGQNGFNIP 910        920        930        940        950 QVTPCPEVGE YLKMSLEDLH SLDSRRIQGC ARRLLCDAYM CMYQSPTMSL |

| |
|---|
| YK |

**SEQ ID NO 5 shows isoform 1 of mouse CYLD protein**

| |
|---|
| 10 20 30 40 50 MSSGLWSQEK VTSPYWEERI FYLLLQECSV TDKQTQKLLK VPKGSIGQYI 60 70 80 90 100 QDRSVGHSRV PSTKGKKNQI GLKILEQPHA VLFVDEKDVV EINEKFTELL 110 120 130 140 150 LAITNCEERL SLFRNRLRLS KGLQVDVGSP VKVQLRSGEE KFPGVVRFRG 160 170 180 190 200 PLLAERTVSG IFFGVELLEE GRGQGFTDGV YQGKQLFQCD EDCGVFVALD 210 220 230 240 250 KLELIEDDDN GLESDFAGPG DTMQVEPPPL EINSRVSLKV GESTESGTVI 260 270 280 290 300 FCDVLPGKES LGYFVGVDMD NPIGNWDGRF DGVQLCSFAS VESTILLHIN 310 DIIPGTSKNI LDQQLKGK |

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: CYLD deletion leads to social deficits, repetitive stereotypic movements and cognitive impairment

As CYLD is highly expressed in neurons and its synaptic protein levels depend on the dosage of the major ASD gene Shank3 (12), the inventors set to understand whether it is involved in brain function by analyzing autism-like behavior of Cyld-/- mice. Cyld-/- mice show normal motor function and anxiety behavior. In detail, Cyld-/- mice did not show any difference compared to controls in the latency to fall from the accelerating rod in the Rotarod, and in total distance travelled in both Open Field (OF) and Elevated Plus Maze (EPM). Moreover, Cyld-/- mice did not exhibit any difference compared to the control animals in the time spent in the different OF arena areas, and in the time spent in both closed and open arms of EPM. Within the social domain, one of the core diagnostic domains of ASD, the inventors detected an impairment in social communication, which in rodents is reflected by calls in the ultrasonic range. The inventors found that in comparison to control mice, Cyld-/- male mice vocalize significantly less, without any difference in the latency to the first call produced (Fig. 1a-b). However, Cyld-/- mice do not show any impairment in both social interaction or social memory in the three-chamber test (Fig. 1c). Importantly, repetitive, stereotypic movements, which represent the second core diagnostic feature of ASD, are also affected in Cyld-/- mice. Cyld-/- mice show an increase in the number of buried marbles in the marble burying test (Fig. 1d) and a reduction in self-grooming behavior compared to controls (Fig. 1e). The third domain the inventors found affected in Cyld-/- mice is cognition. Altered intellectual abilities are a major co-morbid feature of ASD, and to measure these alterations the inventors used the object-recognition test. This test is based on the ability of the animal to memorize an object for a period of 60 minutes, leading to a reduction of exploration time during the second exposure of the same object. The inventors found that indeed the control mice exhibit a significant reduction in exploration time between the first and second exploration (Fig. 1f). In contrast, Cyld-/- mice did not show any difference in exploration time among the two exposures to the same object (Fig. 1f), suggesting a deficit in recognition memory.

### Example 2: CYLD deletion does not modify autism-like behavioral phenotypes in Shank3 deficient mice.

Recently, it has been shown that synaptic CYLD is co-regulated within the brain of Shank3 mutant mice (11, 12). Thus, the inventors crossed Cyld-/- and Shank3-/- mice and analyzed the neurobehavioral phenotype of the resulting Cyld-/-Shank3-/- double mutant mice, in order to clarify if CYLD deletion can modify the autistic phenotype of Shank3-/- mice. The inventors found that similarly to the Cyld-/- mice, also the Shank3 deficient mice or the double mutant animals display an impairment in social communication, showing reduced numbers of calls in presence of a female compared to controls (Fig. 2 a). In contrast, the reduced number of calls was not associated with a change in the latency to the first call emitted (Fig. 2 b). However, the occurrence of repetitive, stereotypic movements affects CYLD and Shank3 deficient mice in opposite ways. Cyld-/- mice show an increase in the number of buried marbles and a reduction in self-grooming behavior, Shank3-/- mice a decreased number of buried marbles and an increased duration of grooming (Fig. 2 c-d), while the double mutant mice performed exactly like Shank3-/- mutant mice in both marble burying test, with a reduction of buried marbles under cage bedding, and in overgrooming (Fig. 2 c-d).

The analysis of cognitive functions with the object-recognition test highlighted an impairment of memorizing an object in Cyld-/-, Shank3-/- and Cyld-/-Shank3-/- mice. Differently from control littermates, the single deficient or double deficient mice did not show a reduction in the exploration time during the second exposure to the same object (Fig. 2 e). To further explore cognitive features, the inventors decided to extend the inventors' analysis using the Morris Water Maze test. The analysis of both escape latency and distance travelled during hidden platform training in the MWM test showed a worse performance of Cyld-/-, Shank3-/- and Cyld-/-Shank3-/- mice in reaching the platform compared to controls, with a significant difference in the distance at day 2 between control and Shank3-/- mice, at day 3 between control and Cyld-/-Shank3-/- mice, and in the latency at day 4 between control and Cyld-/- mice (Fig. 2 f-g). During the probe trial, the inventors did not observe any difference in the percentage of time spent in the target quadrant among the four different genotypes. At the end of the hidden phase training, Cyld-/-, *Shank3-*/*-,* Cyld-/-Shank3-/- and controls reached the same level of performance. In the reversal learning phase, the inventors found that the plasticity needed in order to localize the new place of the hidden platform within the pool was impaired in Cyld-/-, Shank3-/- and Cyld-/-Shank3-/- mice compared to controls. A significant difference in the latency to reach the platform was detectable at day 2 between control and Cyld-/- mice, and at day 3 between control and Shank3-/- mice. At day 3 of reversal learning, the inventors could also detect a significant difference in the distance travelled between Cyld-/-, *Shank3-*/*-,* Cyld-/- Shank3-/- and controls (Fig. 2 h-i), respectively. To sum up, Cyld-/- and Shank3-/- mutants show similar impairments in the social and cognitive domains, but opposite phenotypes in the repetitive domain. Neither phenotype is restored or exacerbated in Cyld-/-Shank3-/- double mutants whose repetitive behavioral profile rather resembles the one of single Shank3-/- mutants.

### Example 3: CYLD function modulates hippocampal plasticity

Dysregulation of neuronal morphology and cytoarchitecture in the hippocampus are typical for ASD (22). Further, a disruption of hippocampal circuits often underlies cognitive impairment, a major co-morbid ASD feature present in Cyld-/- mice. To elucidate a possible dysfunction of CYLD-deficient hippocampal neurons, the inventors performed electrophysiological analysis. Evaluation of Cornu Ammonis 1 (CA1) pyramidal neurons (PNs) showed that the mean amplitude of AMPA-receptor-mediated miniature excitatory post-synaptic currents (mEPSCs) was significantly decreased in Cyld-/- mice when compared to controls, suggesting alterations of basic synaptic properties of CA1 PNs upon loss of CYLD (Fig. 3a-b). No difference was detected in the mean of mEPSCs frequency between Cyld-/- mice and controls (Fig. 3c). Analysis of striatal MSNs was also performed since behavioral tests showed alterations of repetitive stereotypic movements in Cyld-/- mice. However, the analysis of mEPSCs of striatal medium spiny neurons (MSNs) did not show any difference in amplitude and frequency in both dorsal and ventral striatum among Cyld-/- mice and controls. The unaffected basal synaptic properties of striatal MSNs are in line with the unchanged numbers of corticostriatal and thalamostriatal connections, as the inventors did not find any difference among genotypes in the density of pre-synaptic contacts stained with VGluT1 (corticostriatal inputs), VGluT2 (thalamostriatal inputs), or corresponding post-synaptic specializations on MSN dendrites stained with Homer-1, in both dorsal and ventral striatum.

In order to investigate further mechanisms linked to the autism-like phenotypes detected in CYLD-deficient mice the inventors extended the inventor's electrophysiological analysis by measuring neuronal network activity in acute hippocampal slices using Multi-Electrode Array (MEA) recordings. First, the inventors evaluated the excitability of CA3 to CA1 synapses (Schaffer collaterals) in acute slices to understand if the lack of CYLD already implies changes in excitability. One stimulation electrode (filled red dot, Fig. 3 d) was used to stimulate afferent fibers of the Schaffer collaterals to record an input-output relation of CA1 PNs. The amplitudes of field excitatory postsynaptic potentials (fEPSP) (purple circle, Fig. 3d), showed an impaired excitability in Cyld-/- tissue compared to controls (Fig. 3e-f), with a significant difference in the highest stimulation range (4500-5000 mV).

Next, the inventors characterized neuronal long-term synaptic plasticity in Cyld-/- mice by performing classical LTP experiments in CA1. Baseline fEPSPs were electrically induced at an intensity that evoked 30% of the maximum response evoked by input-output curve (1500 mV). Input specificity was controlled by a second independent stimulation (control pathway, empty red circle, see Fig. 3d, grey dots in Fig. 3g). Following 10 min of stable baseline recordings, high frequency stimulation (HFS) was applied for 1 second at a frequency of 100 Hz. The stimulation induced LTP at CA3 to CA1 synapses in control animals, while the level of LTP was much lower in slices from Cyld-/- mice, indicating an impaired hippocampal LTP in mice lacking CYLD (Fig. 3 g). The mean amplitudes of fEPSPs recorded from Cyld-/- mice were significantly reduced 50-60 minutes after LTP-induction when compared to control tissue (Fig. 3h).

### Example 4: CYLD deletion disrupts neuroanatomical properties of CA1 pyramidal neurons (PNs).

Next, the inventors set to investigate whether CYLD deletion affects dendritic tree and spine changes in the young adult brain. To this end, the inventors filled neurons with biocytin and analyzed neuronal and spine morphology. Using IMARIS, the inventors detected a decrease of dendrite total length in CYLD-deficient mice as compared to control animals (Fig. 4a-b), while Sholl analysis of branching complexity did not highlight any significant differences among genotypes (Fig. 4c). Moreover, the inventors counted the number of spines at basal and apical dendrites of CA1 pyramidal neurons and found a significant decrease in the total number of basal spines (Fig. 4d-e) and a trend towards a reduction of apical spines (Fig. 4f) in Cyld-/- mice compared to controls.

In contrast to hippocampal PNs, morphological analysis of biocytin-filled striatal MSNs in the dorsal and ventral striatum, did not highlight differences regarding neuronal dendritic complexity and total dendritic length. Moreover, the total spine number among MSN dendrites was also unchanged within dorsal and ventral striatum of Cyld-/- and control mice.

### Example 5: CYLD deletion results in altered autophagy and mTOR signaling

To elucidate a possible molecular mechanism by which the deubiquitinating function of CYLD is able to regulate the normal protein homeostasis of synapses, the inventors performed biochemical characterization of major PSD proteins that have already been molecularly linked to CYLD (9, 11, 12). To isolate the crude synaptosome fraction, the inventors followed a well-established protocol, which allows the enrichment of synapse-associated proteins (23). In particular, the inventors analyzed by Western blotting Shank3 and PSD-95 protein levels in these fractions from both hippocampus, and striatum, and did not find any change between Cyld-/- mice compared to controls.

Given the electrophysiological impairment in hippocampal CA1 upon loss of CYLD, the inventors further evaluated the levels of the two major subunits of the AMPA receptor, GluA1 and GluA2, both crucial for functional plasticity (24). Interestingly, the amount of GluA1 in the hippocampal synaptosome fraction was significantly higher in Cyld-/- mice compared to controls, while GluA2 protein levels remain unchanged (Fig. 5 a-c). Due to recent evidence implicating CYLD in the autophagic-lysosomal pathway and the fact that autophagy significantly contributes to AMPA receptor internalization (25-27), the inventors decided to analyze changes in autophagy-associated proteins in isolated synaptosomes. Indeed, the quantification of the autophagosome marker LC3B (Microtubule-associated proteins 1A/1B light chain 3B) showed a significant reduction specifically for its lipidated form LC3B-II, but not its un-lipidated form LCB-I, in Cyld-/- hippocampal synaptosomes under steady state conditions (Fig. 5 d-f), indicative for dysregulated autophagic activity. In striatal synaptosomes the inventors did not find any change in both AMPA receptor subunits, GluA1 and GluA2, and in the autophagosome marker LC3B, in line with both electrophysiological and morphological analysis performed.

Mechanistically, autophagosome formation is induced by an upstream blockage of mTOR activity (28). Thus, the inventors investigated whether mTOR signaling was affected in the hippocampus of Cyld-/- mice. Strikingly, the inventors detected a significant increase of total mTOR protein levels, resulting in a significant overactivation of the signaling cascade further downstream reflected by increased p-S6K and p-rpS6 in Cyld-/- mice compared to controls, while total levels of S6K and rpS6 did not change (Fig. 5g-m). It has been shown that K63-specific ubiquitination of mTOR leads to its activation and modulation of the autophagic flux (29). To address the potential function of CYLD in the regulation of mTOR signaling, the inventors further analyzed whether CYLD interacted with the mTOR complex. For this, the inventors specifically immunoprecipitated CYLD and detected mTOR in wild-type hippocampus homogenate by immunoblotting (Fig. 5n), suggesting that CYLD functions as a DUB for mTOR.

### REFERENCES

The references are:
1. G. R. Bignell et al., Identification of the familial cylindromatosis tumour-suppressor gene. Nature genetics 25, 160-165 (2000).
2. P. J. Biggs et al., Familial cylindromatosis (turban tumour syndrome) gene localised to chromosome 16q12-q13: evidence for its role as a tumour suppressor gene. Nature genetics 11, 441-443 (1995).
3. Y. Sato et al., Structures of CYLD USP with Meti- or Lys63-linked diubiquitin reveal mechanisms for dual specificity. Nature structural & molecular biology 22, 222-229 (2015).
4. M. Lork, K. Verhelst, R. Beyaert, CYLD, A20 and OTULIN deubiquitinases in NF-kappaB signaling and cell death: so similar, yet so different. Cell death and differentiation 24, 1172-1183 (2017).
5. G. Mazarei et al., Expression analysis of novel striatal-enriched genes in Huntington disease. Human molecular genetics 19, 609-622 (2010).
6. A. Dosemeci et al., Composition of the synaptic PSD-95 complex. Molecular & cellular proteomics : MCP 6,1749-1760 (2007).
7. A. Dosemeci, S. Thein, Y. Yang, T. S. Reese, J. H. Tao-Cheng, CYLD, a deubiquitinase specific for lysine63-linked polyubiquitins, accumulates at the postsynaptic density in an activity-dependent manner. Biochemical and biophysical research communications 430, 245-249 (2013).
8. S. Thein et al., CaMKII mediates recruitment and activation of the deubiquitinase CYLD at the postsynaptic density. PloS one 9, e91312 (2014).
9. Q. Ma et al., Proteasome-independent polyubiquitin linkage regulates synapse scaffolding, efficacy, and plasticity. Proceedings of the National Academy of Sciences of the United States of America 114, E8760-e8769 (2017).
10. J. Li, Y. Sekine-Aizawa, S. Ebrahimi, S. Tanaka, S. Okabe, Tumor suppressor protein CYLD regulates morphogenesis of dendrites and spines. The European journal of neuroscience 50, 2722-2739 (2019).
11. D. Reim et al., Proteomic Analysis of Post-synaptic Density Fractions from Shank3 Mutant Mice Reveals Brain Region Specific Changes Relevant to Autism Spectrum Disorder. Frontiers in molecular neuroscience 10, 26 (2017).
12. C. Jin et al., Shank3 regulates striatal synaptic abundance of Cyld, a deubiquitinase specific for Lys63-linked polyubiquitin chains. Journal of neurochemistry 150, 776-786 (2019).
13. R. Massoumi, K. Chmielarska, K. Hennecke, A. Pfeifer, R. Fassler, Cyld inhibits tumor cell proliferation by blocking Bcl-3-dependent NF-kappaB signaling. Cell 125, 665-677 (2006).
14. J. Peca et al., Shank3 mutant mice display autistic-like behaviours and striatal dysfunction. Nature 472, 437-442 (2011).
15. K. Radyushkin et al., Complexin2 null mutation requires a 'second hit' for induction of phenotypic changes relevant to schizophrenia. Genes, brain, and behavior 9, 592-602 (2010).
16. J. N. Crawley, Designing mouse behavioral tasks relevant to autistic-like behaviors. Mental retardation and developmental disabilities research reviews 10, 248-258 (2004).
17. A. Thomas et al., Marble burying reflects a repetitive and perseverative behavior more than novelty-induced anxiety. Psychopharmacology 204, 361-373 (2009).
18. A. V. Kalueff et al., Neurobiology of rodent self-grooming and its value for translational neuroscience. Nature reviews. Neuroscience 17, 45-59 (2016).
19. R. Morris, Developments of a water-maze procedure for studying spatial learning in the rat. Journal of neuroscience methods 11, 47-60 (1984).
20. K. Radyushkin et al., Neuroligin-3-deficient mice: model of a monogenic heritable form of autism with an olfactory deficit. Genes, brain, and behavior 8, 416-425 (2009).
21. E. Neher, B. Sakmann, Single-channel currents recorded from membrane of denervated frog muscle fibres. Nature 260, 799-802 (1976).
22. M. Varghese et al., Autism spectrum disorder: neuropathology and animal models. Acta neuropathologica 134, 537-566 (2017).
23. K.-H. Smalla, P. Klemmer, U. Wyneken, "Isolation of the Postsynaptic Density: A Specialization of the Subsynaptic Cytoskeleton" in The Cytoskeleton: Imaging, Isolation, and Interaction, R. Dermietzel, Ed. (Humana Press, Totowa, NJ, 2013), 10.1007/978-1-62703-266-7-11, pp. 265-280.
24. J. M. Henley, K. A. Wilkinson, Synaptic AMPA receptor composition in development, plasticity and disease. Nature reviews. Neuroscience 17, 337-350 (2016).
25. L. Qi et al., CYLD exaggerates pressure overload-induced cardiomyopathy via suppressing autolysosome efflux in cardiomyocytes. Journal of molecular and cellular cardiology 145, 59-73 (2020).
26. M. Shehata, H. Matsumura, R. Okubo-Suzuki, N. Ohkawa, K. Inokuchi, Neuronal stimulation induces autophagy in hippocampal neurons that is involved in AMPA receptor degradation after chemical long-term depression. The Journal of neuroscience : the official journal of the Society for Neuroscience 32, 10413-10422 (2012).
27. H. Shen, H. Zhu, D. Panja, Q. Gu, Z. Li, Autophagy controls the induction and developmental decline of NMDAR-LTD through endocytic recycling. Nature communications 11, 2979 (2020).
28. Y. Wang, H. Zhang, Regulation of Autophagy by mTOR Signaling Pathway. Advances in experimental medicine and biology 1206, 67-83 (2019).
29. J. F. Linares et al., K63 polyubiquitination and activation of mTOR by the p62-TRAF6 complex in nutrient-activated cells. Molecular cell 51, 283-296 (2013).
30. A. Zajicek, W. D. Yao, Remodeling without destruction: non-proteolytic ubiquitin chains in neural function and brain disorders. Molecular psychiatry 26, 247-264 (2021).
31. C. Dobson-Stone et al., CYLD is a causative gene for frontotemporal dementia - amyotrophic lateral sclerosis. Brain : a journal of neurology 143, 783-799 (2020).
32. E. Rosina et al., Disruption of mTOR and MAPK pathways correlates with severity in idiopathic autism. Translational psychiatry 9, 50 (2019).
33. S. C. Borrie, H. Brems, E. Legius, C. Bagni, Cognitive Dysfunctions in Intellectual Disabilities: The Contributions of the Ras-MAPK and PI3K-AKT-mTOR Pathways. Annual review of genomics and human genetics 18, 115-142 (2017).
34. K. D. Winden, D. Ebrahimi-Fakhari, M. Sahin, Abnormal mTOR Activation in Autism. Annual review of neuroscience 41, 1-23 (2018).
35. J. Kim, M. Kundu, B. Viollet, K. L. Guan, AMPK and mTOR regulate autophagy through direct phosphorylation of Ulki. Nature cell biology 13, 132-141 (2011).
36. G. Tang et al., Loss of mTOR-dependent macroautophagy causes autistic-like synaptic pruning deficits. Neuron 83, 1131-1143 (2014).
37. J. Yan, M. W. Porch, B. Court-Vazquez, M. V. L. Bennett, R. S. Zukin, Activation of autophagy rescues synaptic and cognitive deficits in fragile X mice. Proceedings of the National Academy of Sciences of the United States of America 115, E9707-e9716 (2018).

## Claims

1. **A method for diagnosing the presence or an increased risk of developing an autism spectrum disorder, or intellectual Disability (ID),** in a subject, the method comprising: obtaining a nucleic acid from a tissue or body fluid sample obtained from a subject; conducting an assay to identify whether there is a variant sequence, or a plurality of variant sequences, in the subject's nucleic acid; for each variant detected, determining if the variant is a known variant associated with an autism spectrum disorder, or ID, or a previously undescribed variant; if the variant is a previously undescribed variant, determining if the variant is expected to have a deleterious effect on at least one of gene expression and/or protein function; and diagnosing the presence or an increased risk of developing the autism spectrum disorder, or ID, based on the variant sequence or the plurality of variant sequences detected; and wherein at least one of the variant sequences is in at least a portion of *CYLD.*

2. **A method for diagnosing the presence or an increased risk of developing an autism spectrum disorder, or ID,** in a subject, the method comprising: obtaining a biological sample from a tissue or body fluid sample obtained from a subject; conducting an assay to identify whether in the biological sample there is (i) reduced expression of a CYLD gene, or (ii) reduced activity and/or stability of a CYLD protein; and wherein such reduced expression in (i) and/or reduced activity and/or stability in (ii) is indicative for the presence or an increased risk of developing an autism spectrum disorder, or ID, in the subject.

3. The method of claim 2, wherein the biological sample is from a central nervous system of the subject, preferably is a brain sample.

4. **A method for identifying mutations correlated with the presence or increased risk of developing an autism spectrum disorder, or ID,** the method comprising: identifying a nucleic acid to be evaluated as having a sequence that if mutated may be or is associated with the development of autism; obtaining a nucleic acid sample from a tissue or body fluid sample obtained from a subject having an autism spectrum disorder, or ID; and conducting an assay to identify whether there is a mutation in the nucleic acid sequence in the subject having autism as compared to the nucleic acid sequence in individuals who do not have an autism spectrum disorder, wherein the presence of the mutation in a subject with an autism spectrum disorder, or ID, indicates that the mutation may be associated with the development of the autism spectrum disorder, or ID, wherein the nucleic acid sequence for which the presence or absence if a mutation is evaluated is at least a portion of the CYLD gene.

5. The method of claim 4, further comprising determining if the mutation is expected to have a deleterious effect on at least one of gene expression and/or protein function.

6. The method of claim 4, further comprising determining if the mutation has an effect of on enzymatic activity of CYLD protein to remove Lysine 63 (K63)-linked polyubiquitin chain from a substrate protein.

7. The method of any one of claims 1 to 6, wherein the autism spectrum disorder, or ID, is a neuropsychiatric condition that causes severe and pervasive impairment in thinking, feeling, language, and in social ability (ability of a subject to relate to others), and is specifically selected from autistic disorder, autism, pervasive development disorder not otherwise specified (PDD-NOS), Asperger syndrome, Rett syndrome and childhood disintegrative disorder.

8. **A use of an isolated nucleic acid in the method of any one of claims 1 to 7,** wherein the isolated nucleic acid comprises a sequence or a variant sequence from a CYLD gene (preferably human CYLD), wherein the variant sequence comprises a genetic variant that is indicative of an autism spectrum disorder, or ID.

9. **A method for screening compounds or compositions for a modulator of a neuropsychiatric manifestation associated with an autism spectrum disorder, or ID,** comprising the steps of:
(a) Bringing into contact a candidate compound or composition with (i) a CYLD protein and/or (ii) a CYLD nucleic acid;
(b) Determining in (i) an activity and/or stability of the CYLD protein in presence and in absence of the candidate compound or composition; and/or determining in (II) a protein- or mRNA-expression from the CYLD nucleic acid, or a stability of the CYLD nucleic acid, in presence and in absence of the candidate compound or composition;
Wherein, as determined in (i), an increased or reduced activity and/or stability of the CYLD protein in presence compared to absence of the candidate compound or composition, and/or wherein, as determined in (ii), an increased or reduced protein- or mRNA-expression from the CYLD nucleic acid, or an increased or reduced stability of the CYLD nucleic acid, in presence compared to absence of the candidate compound or composition, indicates the candidate compound or composition as a modulator of a neuropsychiatric manifestation associated with an autism spectrum disorder, or ID.

10. **An in-vivo method for screening compounds or compositions for a modulator of a neuropsychiatric manifestation associated with an autism spectrum disorder, or ID,** comprising the steps of:
(a) Administering to a non-human animal a candidate compound or composition;
(b) Determining (quantifying) in the non-human animal at least one neuropsychiatric manifestation associated with an autism spectrum disorder, or ID, compared to a non-human animal that did not receive the candidate compound or composition;
Wherein, as determined in (b), an increased or reduced neuropsychiatric manifestation associated with an autism spectrum disorder, or ID, in the non-human animal that received the candidate compound or composition compared to the non-human animal that did not receive the candidate compound or composition indicates the candidate compound or composition as modulator of a neuropsychiatric manifestation associated with an autism spectrum disorder, or ID.

11. The method of claim 9 or 10, wherein the non-human animal is **characterized by** a reduced expression, function and/or stability of CYLD protein, such as a CYLD genetic knock-out or knock-down (RNAi) animal, and wherein the modulator to be screened is an antagonist of a neuropsychiatric manifestation associated with an autism spectrum disorder, or ID.

12. **A compound or composition for use in the treatment of an autism spectrum disorder, or ID,** in a subject, wherein the compound or composition is a CYLD protein or a variant or fragment thereof, or is a CYLD nucleic acid suitable for the expression of the CYLD protein or variant or fragment thereof.

13. The compound or composition for use of claim 12, wherein the treatment involves an administration of the compound or composition to a central nervous system (CNS) of the subject.

14. The compound or composition for use of claim 13, wherein the treatment involves an administration to a postsynaptic neuron in the CNS of the subject.

15. **A genetically modified non-human animal,** preferably a mouse or rat, wherein the transgenic non-human animal comprises at least one genetic mutation within the endogenous CYLD locus, and / or at least one recombinant genetic construct that modulates expression, function and / or stability of a CYLD protein, preferably within a central nervous system of the non-human animal.
